Europäisches Patentamt

European Patent Office

Office européen des brevets

(11) Publication number: 0 360 329
A1

# EUROPEAN PATENT APPLICATION

(21) Application number: 89202292.2

(22) Date of filing: 08.09.89

(51) Int. Cl.⁵: A61M 5/50

(30) Priority: 13.09.88 NL 8802248

(43) Date of publication of application:
28.03.90 Bulletin 90/13

(84) Designated Contracting States:
AT BE CH DE ES FR GB GR IT LI LU NL

(71) Applicant: KONINKLIJKE EMBALLAGE
INDUSTRIE VAN LEER B.V.
Amsterdamseweg 206
NL-1182 HL Amstelveen(NL)

(72) Inventor: Stegeman, Bernardus Hendrikus
Marinus
Claude Monetstraat 4
NL-1506 KB Zaandam(NL)

(74) Representative: de Bruijn, Leendert C. et al
Nederlandsch Octrooibureau
Scheveningseweg 82 P.O. Box 29720
NL-2502 LS 's-Gravenhage(NL)

(54) Hypodermic syringe.

(57) Hypodermic syringe, having a cylinder (1), a piston (5) in said cylinder and a piston rod (6) with means for moving the piston towards or away from the needle (3) attached at one end of the cylinder. The invention being formed by an element on the piston rod, which is slidable along the piston rod in only one direction, towards the piston, and which engages the inner wall of the cylinder such that said element can only slide along said inner wall towards the needle. In its most simple form said element is a plate placed over a rib of a piston rod with a section in the form of a across, which plate has outer points which engage the iner wall and inner points which engage said rib.

Fig-2

## Hypodermic syringe

The invention relates to a hypodermic syringe, comprising a cylinder which at one end has a bottom with a passage to a hypodermic needle, a piston or similar element which fits slidably into the cylinder, a piston rod which is attached to the piston or forms part thereof, and means on the cylinder or piston for displacing these parts relative to each other.

Such a hypodermic syringe is generally known.

In order to avoid resterilization of hypodermic syringes, it has already been known for a long time to use hypodermic syringes which due to their simple and cheap design and the choice of material for them can essentially be used only once and are subsequently thrown away. They are not suitable for cleaning again and sterilizing.

There is, however, a risk of such known hypodermic syringes being used more than once, which can give rise to infection, for example in the case of drugs users.

DE-A-2.354.628 discloses a hypodermic syringe which has a facility making it possible to limit the use of the hypodermic syringe to a single time through the fact that at the end of the injection stroke the wall of the cylinder is perforated by a part fitted on the piston. Owing to this perforation at the end of the stroke, the hypodermic syringe cannot be used any more.

This does not, however, provide a full guarantee that it will not be re-used, for the piston can be returned to its initial position. If the perforation is closed by holding it closed or sealing it, then re-use is possible again. Moreover, there is also a possibility of re-use if the piston does not move far enough in the direction of the bottom for the perforation to take place.

The object of the invention is to produce a hypodermic syringe in the case of which re-use is ruled out. The object of the invention is also to achieve this in a very simple manner, also including the possibility of converting existing hypodermic syringes in a simple manner into hypodermic syringes according to the invention.

This object is achieved according to the invention in the first place in that an element is placed on the piston rod in such a way that this element is slidable along the piston rod only towards the piston, and this element acts on the inside wall of the cylinder in such a way that the element is slidable only towards the bottom of the cylinder along this inside wall.

Since the piston rod is slidable relative to the element in only one direction, i.e. in a direction in which element and piston are moved towards each other, it is possible to fill the hypodermic syringe by carrying out a suction stroke with the piston until the piston runs against the element. When the piston with the piston rod is subsequently moved in the direction of the bottom of the cylinder, then the element is taken along with it, and slides along the cylinder wall in the process, but cannot be moved back.

Therefore, once the piston has reached the end of its stroke, either a full or a partial stroke, and an attempt is made to move the piston back, this is out of the question, because the element prevents it and forms a stop for the piston. The condition here is that the filling stroke is actually carried out fully up to the stop.

An important advantage of the solution according to the invention is also that, in addition to re-use being ruled out, the position of the element on the piston rod is the determining factor for the maximum quantity which can be injected with the hypodermic syringe. The usual volumes are 2, 5 or 10 cc, which can now be set with the invention.

The manufacturer will position the element in such a way that the filling stroke is slightly greater than the nominal volume per type of syringe, or differently if desired.

As a precaution, the user can in certain cases set a reduced stroke volume beforehand. This is simple to do by observing the displacement of the element if the piston rod is moved lightly to and fro with the piston near the bottom of the cylinder. Each time it is pressed the element moves in the direction of the piston, until the desired position for the reduced stroke volume is achieved.

The principle on which the invention is based can be achieved in many ways.

For example, it is possible for the piston rod and the element to act on each other with friction faces which permit relative displacement in one direction, but which block it in the opposite direction. This can be achieved by, for example, making the element from an elastic friction material, such as rubber, which through deformation and expansion blocks movement in one direction, but allows it in the other direction.

It is also possible for the cylinder wall and the element to act on each other by means of friction faces which allow relative displacement in one direction, but block it in the opposite direction. This again can be achieved in the same way as specified above. The two possibilities can be combined.

Another possibility is that the element acts on the piston rod with a saw tooth profile and this saw tooth profile can be a file tooth profile, in particular a file tooth profile of great fineness which has the ability to dig into the material of rod or cylinder wall in one direction of movement and to glide over them in the other direction. Here too, a comparison with the differences in friction has to be made.

However, many simpler solutions are conceivable.

For example, it is possible according to the invention in a hypodermic syringe whose piston rod has the common cross-shaped cross-section to make the element from an L-shaped plate which rests with the legs against two faces of the cross-shaped section of the piston rod standing at right angles to each other, said L-shaped plate having at the ends of the legs a point which forms an acute angle with the cylinder wall, opening out towards the bottom of the cylinder, while near the angle between the legs the plate has a sharp edge which is bent out towards the corner of the section, and which forms an acute angle with this face whose opening faces the piston.

Nothing need then be changed in the most common hypodermic syringes. The envisaged effect is obtained by placing the L-shaped plate in the quadrant between two legs of the cross-shaped section standing at right angles to each other.

According to the invention the element can be a spring which is clamped between cylinder wall and piston rod and acts with a sharp edge on the piston rod and acts with a sharp outer edge on the inside wall of the cylinder. If the piston rod has a cross-shaped cross-section, then at least one leg of the cross-shaped cross-section can be designed with an elongated slit in the centre dividing plane of said leg, said slit having positioned in it a wire spring which acts by means of sharp points facing in opposite directions at an acute angle on piston rod or cylinder wall. This spring can have outward-curved legs with sharp points, but can also be a hairpin spring whose ends cross each other. In both cases the effect envisaged by the invention will occur if the spring is correctly positioned.

The piston rod with cross-shaped cross-section is very commonly used, but if the piston rod has a cylindrical cross-section which is known per se, then the spring can be a V-shaped Belleville spring which acts with a sharp inner or outer edge on piston rod or cylinder wall. The cylinder wall must be smooth along the part mating with the piston. The piston rod can, however, have a toothed profile.

According to another embodiment, in which the piston rod again has a cross-shaped cross-section, according to the invention a block can be placed in at least one of the quadrants of the profile of the rod, said block being provided with a spring-loaded tooth which is directed obliquely at the cylinder wall at an angle with the wall which opens out towards the cylinder bottom, and said block having a tooth which acts on a leg of the rod. Block and profile can thus engage with each other in a toothed fashion.

The spring can, however, be formed by the earlier-mentioned wire spring, which then is not placed in a slit, but is placed in the block and has resilient sharp points projecting therefrom.

It is preferable to have an embodiment in which the piston rod also has a cross-shaped cross-section, and in which the spring is a leaf which is folded V-shaped over a rib of the rod, and the legs of which are flanged outwards in a plane at right angles to the rib on which the spring is placed, while the distance between the opposite ends of the flanged parts of the legs of the leaf when unstressed is greater than the distance between the opposite parts of said cylindrical inside wall, viewed in the plane of the flanged leg ends. Such a spring is easy to make and easy to fit, and can thus be used without further ado in existing hypodermic syringes which have a piston rod of cross-shaped cross-section.

The outside edges of the flanged legs of this spring can form an acute angle with the cylinder wall which opens out towards the bottom. The V-shaped part of the spring can have lips which are pressed inwards and act on the rib, and these lips acting on the rib are then at an acute angle which opens out towards the bottom. Such an element can be placed astride a rib of the cross-shaped section at the desired distance from the piston, and both can subsequently be slid into the cylinder until the piston has reached the bottom. The piston can then be withdrawn, while the element remains in place, permitting a predetermined quantity to be drawn into the hypodermic syringe, this quantity thus being determined by the position of the element on the piston rod. When an injection is subsequently given the element moves along with the piston rod and blocks any return movement. The sharp edges or points acting on the cylinder wall used in the various embodiments can be designed in such a way here that they perforate or damage said wall as soon as an attempt is made to force the piston back.

The invention will now be explained in greater detail with reference to the drawings.

Fig. 1 shows schematically the hypodermic syringe according to the invention in cross-section, in the initial position.

Fig. 2 shows the filling of the hypodermic syringe of Fig. 1.

Fig. 3 shows the position near the end of the stroke intended for injection of the liquid.

Fig. 4 shows a part of Fig. 3 on a larger scale.

Fig. 5 serves to illustrate the assembly.

Fig. 6 shows the spring of the hypodermic syringe according to the invention on a larger scale.

Fig. 7 is a cross-section along the line VII-VII of Fig. 6.

Fig. 8 is a longitudinal section through a different embodiment.

Fig. 9 is a cross-section along the line IX-IX of Fig. 8.

Fig. 10 shows yet another embodiment in longitudinal section; and

Fig. 11 shows the cross-section thereof along the line XI-XI of Fig. 10.

Fig. 12 shows a further variant in longitudinal section.

Fig. 13 shows a variant of the embodiment of Fig. 12, in longitudinal section; and

Figs. 14 and 15 show, finally, another variant in longitudinal section and cross-section respectively.

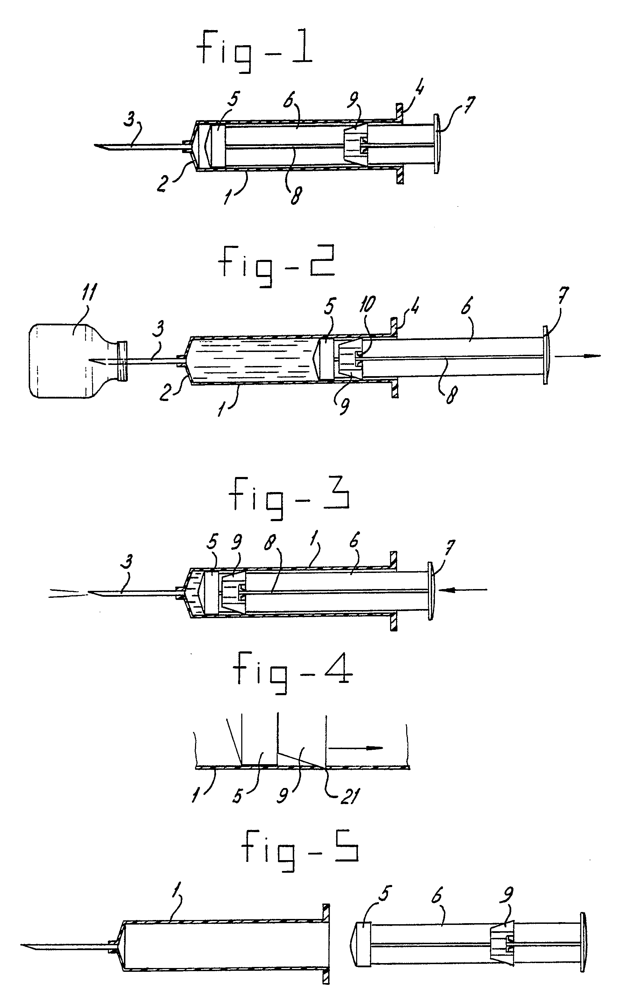

Figs. 1, 2 and 3 show a hypodermic syringe, comprising a cylinder 1 having at one end a bottom 2 in which a needle 3 can be fitted or fixed on a conical end (not shown) thereof. The cylinder also has a flange 4.

Inside the cylinder is a piston 5 with a piston rod 6 of cross-shaped cross-section, which is provided at the end with an operating button 7.

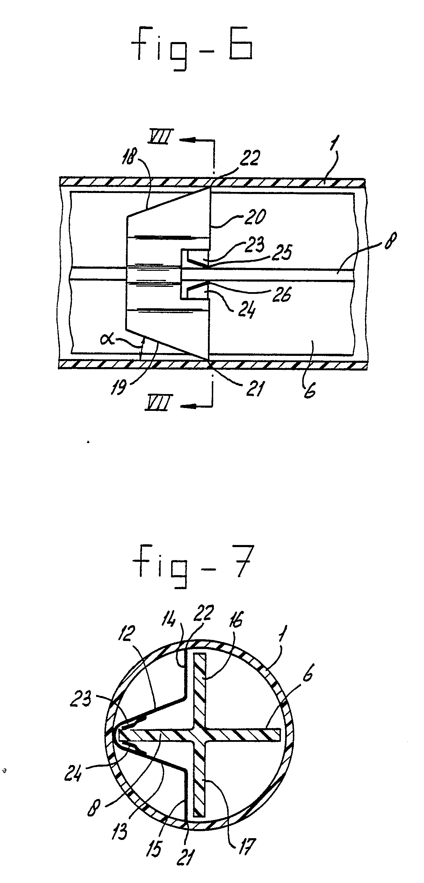

The rib 8 of the piston rod has placed on it a spring 9, the details of which can be seen in Figs. 6 and 7. This spring 9 grips the rib 8 of the piston rod in such a way that, as shown in Fig. 2, the piston rod can move through the grip with the lips 10, in such a way that the piston is moved towards the spring 9 for filling of the cylinder via the needle from an ampoule 11.

When an injection is being given, as shown in Fig. 3, the spring 9 is carried along by the piston rod 6. Spring and piston thus remain right next to each other. When the piston reaches the end of its stroke it can be moved back only until it touches the spring 9, which locks itself in the wall of the cylinder 1.

As shown in Figs. 6 and 7, the spring is made up of a leaf bent in a V-shape, the V-shaped part of which is indicated by the legs 12 and 13 whose ends 14 and 15 are flanged in a plane at right angles to the rib 8, and thus parallel to the ribs 16 and 17 standing at right angles thereto.

The outside edges 18 and 19 of these flanged parts 14 and 15 form an acute angle alpha with the inside wall of the cylinder 1, and thus form a sharp point 21, 22 with the rear edge 20.

The V-shaped legs 12 and 13 have lips 23, 24 which are pressed inwards out of the face, and which with a sharp edge 25, 26 act on the respective side faces of the rib 8.

If an attempt is made to move the spring in the opposite direction, then the point 21, 22 will cut through the wall of the cylinder 1 and thus perforate it, as shown in Fig. 4.

This perforation is not necessary, for the blocking has already been achieved beforehand.

Fig. 5 shows, finally, that prior to the placing of the piston 5 in the cylinder 1 the spring 9 can be clamped on the piston rod at any desired distance from the piston. The distance from the piston then determines how far the piston can be moved outwards for the filling stroke shown in Fig. 2.

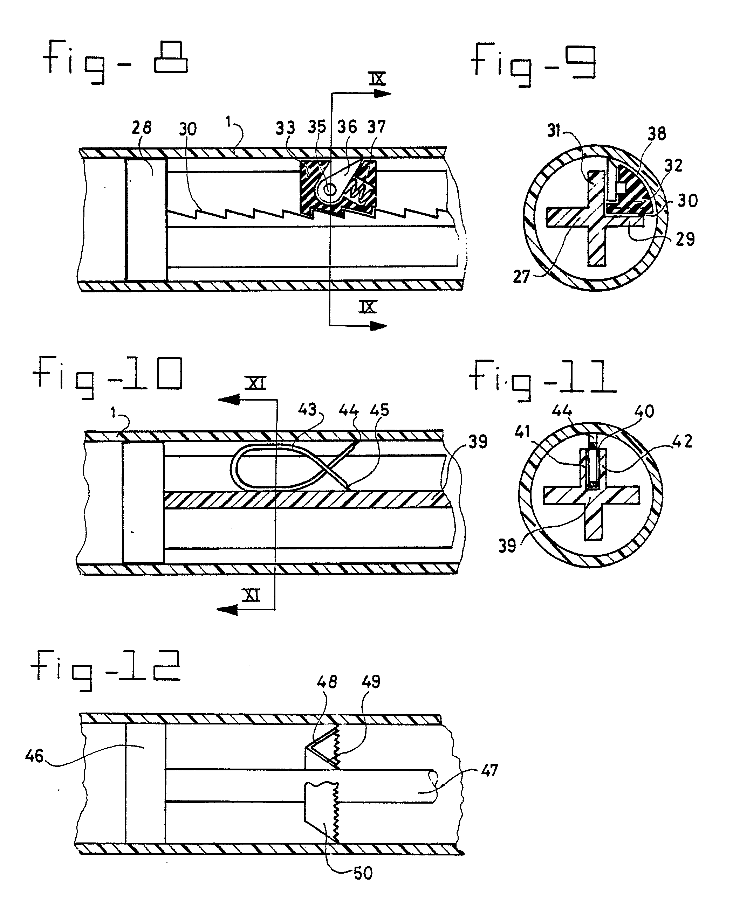

In the embodiment of Figs. 8 and 9 the piston rod 27 of the piston 28 has a cross-shaped cross-section.

One of the legs or ribs 29 of this cross-shaped section is provided on its surface with toothing 30. In the quadrant situated between the leg 29 and the leg 31 standing at 90° thereto is a block 32 which with a toothed face 33 engages in the toothing 30. This block is provided with a tooth 36 which is hingedly fixed at 35, and on which a spring 37 acts.

This block has a slight clearance 38 relative to the inside wall of the cylinder 1, which makes it possible to lift the block out of the toothing. This means that it can be slid to the left in Fig. 8, but not to the right. During this sliding the tooth 36 is pressed inwards.

Displacements of the piston with piston rod and the block to the right in Fig. 8 are prevented by the tooth, because it then penetrates into the wall of the cylinder, the material of which is generally made of plastic.

In the variant shown in Figs. 8 and 9 the piston rod must therefore be adapted by providing the toothing.

In the embodiment according to Figs. 1 to 7 this was not necessary.

In the embodiment according to Figs. 10 and 11 the piston rod is also cross-shaped in cross-section. One of the legs of the cross is designed with a slit 40 running along the entire length, and thus has in fact two cheeks 41, 42 running parallel to each other. This slit contains a hairpin-type wire spring 43 whose ends lie across each other and have sharp points 44, 45. The point 44 acts on the inside wall of the cylinder 1; point 45 on the bottom of the slit 40. The two sharp wire ends or points are placed at such an angle that displacement is possible in one direction along the face with which they are in contact, and not in the opposite direction.

Of course, it is also possible to design the hairpin spring with legs which do not cross each other, but run essentially parallel to each other and have outward-flanged points.

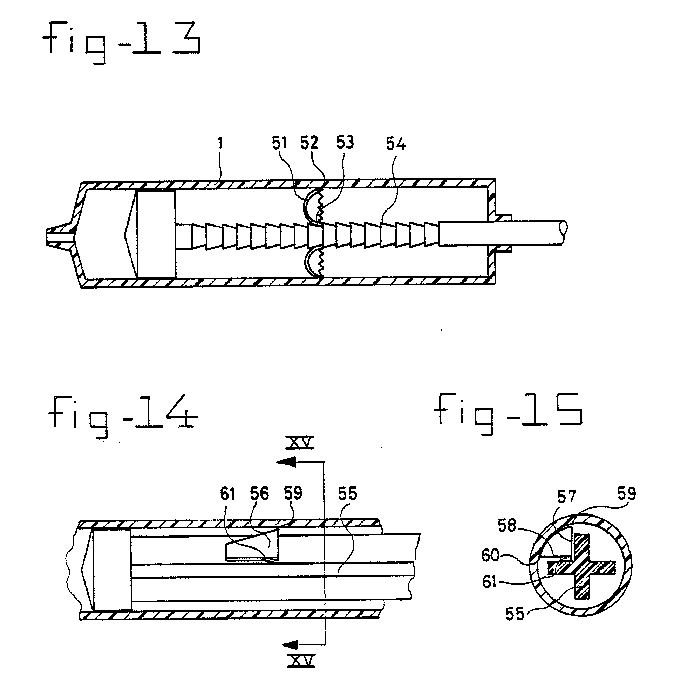

In the embodiment of Fig. 12 the piston 46 has a cylindrical piston rod 47. In this embodiment the element is a V-shaped Belleville spring 48, which with a sharp edge 49 acts on the piston rod and with a sharp or toothed edge 50 acts on the cylinder wall.

In the embodiment of Fig. 13 a Belleville spring 51 is also used, acting with a sharp edge 52 on the cylinder wall 1, but the inside edge 53 of the Belleville spring engages in a toothing 54 of the piston rod, which piston rod can have a circular cross-section, but can also have a more angular cross-section.

In the embodiment of Figs. 14 and 15 the piston rod again has a normal cross-shaped cross-section, and this piston rod 55 thus does not differ from a piston rod of the type shown in Figs. 1 to 7.

In this embodiment the element is a metal plate bent in an L-shape, said plate 56 having a vertical leg 57 and a horizontal leg 58, and these legs having a sharp point 59, 60 respectively. This plate 56 is preferably slightly clamped in a quadrant between the legs of the cross-shaped cross-section standing at right angles to each other, and thus acts with the points 59 and 60 on the cylinder in such a way that displacement towards the bottom of the cylinder, but not in the opposite direction, is possible.

The one-way freedom of movement relative to the piston rod is obtained by means of a lip 61 which is bent out of the face of the leg 58 and which also acts with a sharp edge or point on the face of a leg of the cross-shaped piston rod.

All of the embodiments described above have the feature that the initial distance between the element and the piston determine how much liquid can be drawn into the hypodermic syringe, and the feature that after injection the piston cannot be used again to fill up the hypodermic syringe, due to the fact that its freedom of movement is blocked. In the embodiments according to Figs. 8 to 13 modifications of the existing design are necessary. In the embodiments according to Figs. 1 to 7 and 14, 15 this is not the case.

## Claims

1. Hypodermic syringe, comprising a cylinder which at one end has a bottom with a passage to a hypodermic needle, a piston or similar element which fits slidably into the cylinder, a piston rod which is attached to the piston or forms part thereof, and means on the cylinder or piston for displacing these parts relative to each other, characterized in that an element is placed on the piston rod in such a way that this element is slidable along the piston rod only towards the piston, and this element acts on the inside wall of the cylinder in such a way that the element is slidable only towards the bottom of the cylinder along this inside wall.

2. Hypodermic syringe according to Claim 1, characterized in that the piston rod and the element act on each other with friction faces which permit relative displacement in one direction, but which block it in the opposite direction.

3. Hypodermic syringe according to Claim 1 or 2, characterized in that the cylinder wall and the element act on each other by means of friction faces which allow relative displacement in one direction, but block it in the opposite direction.

4. Hypodermic syringe according to Claim 1, 2 or 3, characterized in that the element acts on the piston rod with a saw tooth profile.

5. Hypodermic syringe according to Claim 4, characterized in that the saw tooth profile is a file tooth profile.

6. Hypodermic syringe according to Claim 1, in which the piston rod has a cross-shaped cross-section, characterized in that the element is an L-shaped plate which rests with the legs against two faces of the cross-shaped section of the piston rod standing at right angles to each other, said L-shaped plate having at the ends of the legs a point which forms an acute angle with the cylinder wall, opening out towards the bottom of the cylinder, while near the angle between the legs the plate has a sharp edge which is bent out towards the corner of the section, and which forms an acute angle with this face whose opening faces the piston (Fig. 14, 15).

7. Hypodermic syringe according to Claim 1, characterized in that the element is a spring which is clamped between cylinder wall and piston rod and acts with a sharp edge on the piston rod and acts with a sharp outer edge on the inside wall of the cylinder (Fig. 12, 13).

8. Hypodermic syringe according to Claim 7, in which the piston rod has a cross-shaped cross-section, characterized in that the spring is a leaf which is folded V-shaped over a rib of the rod, and the legs of which are flanged outwards in a plane at right angles to the rib on which the spring is placed, while the distance between the opposite ends of the flanged parts of the legs of the leaf when unstressed is greater than the distance between the opposite parts of the cylindrical inside wall, viewed in the plane of the flanged leg ends (Figs. 1 to 7).

9. Hypodermic syringe according to Claim 8, characterized in that the outside edges of the flanged legs of the spring form an acute angle with the cylinder wall which opens out towards the bottom.

10. Hypodermic syringe according to Claim 8 or 9, characterized in that the V-shaped part of the spring has lips which are pressed inwards and act on the rib.

11. Hypodermic syringe according to Claim 10, characterized in that the lips act on the rib at an acute angle which opens out towards the bottom.

12. Hypodermic syringe according to Claim 7, in which the piston rod has a cross-shaped cross-section, characterized in that at least one leg of the cross-shaped section is designed with an elongated slit in the centre dividing plane of said leg, said slit having positioned in it a flat wire spring, such as a hairpin spring, which acts by means of sharp points facing in opposite directions at an acute angle on the piston rod or cylinder wall.

13. Hypodermic syringe according to Claim 12, characterized in that the spring is a hairpin spring whose legs cross each other (Fig. 10).

14. Hypodermic syringe according to Claim 7, in which the piston rod has a cylindrical cross-section, characterized in that the spring is a V-shaped Belleville spring (Fig. 12).

15. Hypodermic syringe according to Claim 1, in which the piston rod has a cross-shaped cross-section, characterized in that a block is placed in at least one of the quadrants of the profile of the rod, said block being provided with a spring-loaded tooth which is directed obliquely at the cylinder wall at an angle with the wall which opens out towards the cylinder bottom, and said block having a tooth which acts on a leg of the rod.

16. Hypodermic syringe according to Claim 15, characterized in that block and profile engage with each other in tooth form.

Fig-1

Fig-2

Fig-3

Fig-4

Fig-5

Fig-6

Fig-7

fig-8

fig-9

fig-10

fig-11

fig-12

fig-13

fig-14

fig-15

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | EP-A-0 308 040 (PATH)<br>* Whole document * | 1-4,6,7 | A 61 M 5/50 |
| P,A | | 5,10-12 | |
| A | US-A-4 731 068 (HESSE)<br>* Figures 1-8,11-23; column 1, lines 25-50 * | 1 | |
| A,D | DE-A-2 354 628 (CHIQUIAR ARIAS)<br>* Figures 1,2,4,5 * | 1 | |
| A | AU-B- 16 859 (DEAN)<br>* Figures 1,2; column 3, lines 29-51 * | 7 | |
| A | EP-A-0 225 439 (DENTSPLY INTERNATIONAL INC.)<br>* Figures 1,3-6; abstract * | 15,16 | |

TECHNICAL FIELDS
SEARCHED (Int. Cl.5)

A 61 M

The present search report has been drawn up for all claims

| Place of search | Date of completion of the search | Examiner |
|---|---|---|
| THE HAGUE | 14-12-1989 | SEDY, R. |